# EUROPEAN PATENT APPLICATION

(11) **EP 3 329 942 A2**
(43) Date of publication of application: **06.06.2018**
(21) Application number: 18152632.8
(22) Date of filing: 14.03.2014
(51) Int. Cl.: A61K 49/00

(54) **QUANTUM DOTS FOR DIAGNOSTIC IMAGING**

(30) Priority: 15.03.2013 US 201361798753 P
(62) Divisional of application: 14747122.1
(71) Applicant: Nanoco Technologies Ltd, Manchester M13 9NT (GB)
(72) Inventor: Gresty, Nathalie, Chester, CH3 5HG (GB)
(74) Representative: Dauncey, Mark Peter

(57) **Abstract**

Methods for detecting disease in a patient are disclosed. The methods involve administering to the patient a quantum dot-analyte conjugate, which includes an analyte that binds to a marker for the disease in the patient's gastrointestinal tract. The analyte is conjugated to a quantum dot having a characteristic emission wavelength. Using an endoscopic modality, a physician can illuminate portions of the patient's gastrointestinal tract and detect the presence of the marker based on emission of the quantum dot. Also disclosed are methods of predicting a response to a treatment in a patient.

## Description

### BACKGROUND

### 1. Field of the Invention.

The invention relates to diagnostic imaging for medical applications, and more particularly, to the use of quantum dots (QDs) for diagnostic imaging.

### 2. Description of the Related Art including information disclosed under 37 CFR 1.97 and 1.98.

For the detection of conditions affecting the small bowel, a number of diagnostic imaging techniques are known in the prior art. Existing techniques for diagnosis of diseases affecting the small bowel include endoscopic techniques to view the bowel internally, and radiological imaging techniques. The attributes and limitations of existing imaging techniques are often depended on the location and extent of inflammation in the small bowel.

### Endoscopic Techniques

Conventional endoscopic techniques can be used to visualise the upper gastrointestinal (GI) tract (oesophagus, stomach and duodenum) using an 'upper GI endoscopy' or 'gastroscopy' and the lower bowel (colon and terminal ileum) using a 'colonoscopy'. Advantages of the procedures include that biopsies can be taken, however there is a large area of the small bowel, *i.e.* the jejunum and the proximal ileum, that cannot be viewed. Further, the procedure is invasive and uncomfortable for the patient, often requiring sedation.

Longer endoscopes to view and biopsy the entire small bowel have been developed, including 'push endoscopy' and 'double-balloon enteroscopy' techniques. However, these diagnostic methods are not widely available and are unfavourable due to their requiring long periods of sedation (up to approximately three hours).

Capsule endoscopy is a more recent technique to image the entire small bowel. For example, the PillCam SB (Given® Imaging) video capsule is the size of a large vitamin capsule (11 mm x 26 mm), which the patient swallows to take 50,000 images of the bowel over an eight hour period. The images are recorded on a sensor belt, worn around the patient's waist. Advantages of the procedure include that it is non-invasive; the capsule is designed to travel through the digestive system and be excreted with a bowel movement. However, complications can occur when there is structuring of the bowel, in which case the capsule can become stuck and may require surgical removal. Thus, the technique is not suitable for all patients. A patency capsule can be administered to identify those patients at risk of capsule retention. A further disadvantage is that capsule endoscopy cannot provide histological information. Thus, there is a need for a method to determine the underlying cause of abnormalities observed during capsule endoscopy, as an alternative to a biopsy in a conventional endoscopy.

### Radiological Imaging Techniques

Radiological imaging techniques can be used to study structural abnormalities in the small bowel, however they cannot provide histological information therefore their diagnostic value is limited. The choice of imaging technique may depend on the patient's symptoms, previous exposure to radiation and the availability of imaging resources.

Ultrasound scanning is a readily accessible imaging technique that is safe to use during pregnancy. Though abdominal ultrasound can be useful to eliminate possible other diagnoses that can cause abdominal pain, *e.g*. liver and kidney problems, gallstones, *etc.,* its use in small bowel imaging is limited. A skilled radiologist may be able to use ultrasound to distinguish between structuring caused by active inflammation and that as a result of scar tissue, however its diagnostic potential is limited.

Plain abdominal X-ray imaging is also readily available. Its use in bowel imaging is very limited but it can be used to show areas of obstruction (a medical emergency). To effectively image the small bowel, a contrast agent must be administered. A barium follow-through examination uses barium sulphate, a non-toxic, radiopaque compound that appears white on an X-ray, to image the small bowel. The patient drinks the barium sulphate preparation, then X-rays are taken periodically as the contrast agent passes through the bowel. As the barium approaches the end of the small bowel, a portable X-ray machine is used to follow the transit of the barium in real-time. The patient may be asked to change positions and implements may be used to compress the abdomen, to attempt to separate out adjacent loops of bowel. Disadvantages of the technique include that it is a lengthy procedure, which subjects the patient to radiation for extended periods of time (typically the radiation dose is equivalent to that of 100 plain abdominal X-rays, or a year's worth of background radiation exposure). The barium sulphate can be unpalatable to drink. Further, in patients with a low body mass index, which is a prominent feature of small bowel diseases, it can be difficult to compress the abdomen sufficiently to distinguish between loops of bowel, rendering the technique redundant.

Computer tomography (CT) scanning uses X-rays to provide cross-sectional images of the abdomen, including the small bowel. The faster acquisition times can provide much clearer images than those obtained from magnetic resonance imaging (MRI) scans. However, the technique exposes the patient to high doses of ionising radiation.

Labelled white blood cell scanning studies the aggregation of white blood cells in the body to locate sites of inflammation. A sample of the patient's blood is taken, then the leukocytes are extracted by centrifugation and labelled with a radioisotope, ^{99m}Tc. The white blood cells are injected back into the patient's blood, then the patient is scanned, periodically, with a gamma camera to assess whether the leukocytes accumulate in a specific part of the body; this can be an indication of the location of inflammation. The technique is also useful to distinguish between active inflammation and symptoms arising from scar tissue, since scarring will not show up on a labelled white blood cell scan. However, the technique does not distinguish between different causes of inflammation, *e.g*. infection or auto-immune, therefore it is of limited diagnostic benefit. Due to the high level of exposure to radiation and with improvements in techniques such as magnetic resonance imaging (MRI) scanning, labelled white blood cell scans are now rarely requested by gastroenterologists.

Recent advances in MRI scanning have made it a more prominent technique in the diagnosis of small bowel conditions. Since it uses radiowaves, non-ionising radiation, it is safe for use during pregnancy and where patients have previously been exposed to high doses of radiation. The technique uses powerful magnets, inducing paramagnetic nuclei in the body, *e.g*. water molecules, to align with the magnetic field, while a radio frequency current produces an electromagnetic field to systematically alter the direction of the magnetic field. Water molecules relax to their equilibrium positions at different rates, depending on the tissue in which they are located. Contrast agents can be administered intravenously and/or orally to alter the relaxation times of the water molecules. Thus, the technique can be used to distinguish between different types of tissue and sites of inflammation. However, one of the challenges for abdominal imaging is that the muscles in the bowel wall are constantly contracting, which can reduce image clarity compared to that of CT scans where the acquisition time is much shorter. Muscle relaxants can be administered to reduce the problem. MRI is not, however, suitable for all patients; since the scanners use strong magnets, patients with metal pins or plates, *etc.* and metal workers who may have inadvertently taken in metal fragments should not be scanned. Further, oral contrast agents can require the patient to drink several litres of fluid, which may not be feasible if they are suffering from bowel obstruction of vomiting symptoms. For patients suffering from claustrophobia, MRI scanning may be contraindicated.

In summary, a vast selection of small bowel diagnostic techniques are available, however only a small number are capable of observing microscopic changes in the bowel to accurately distinguish between different causes of inflammation. Of those available, only push or double-balloon enteroscopy techniques have the potential to view the entire small bowel, methods which are not widely available and require extended periods of sedation. Thus, there is a need for a minimally invasive imaging technique that is capable of imaging the entire small bowel, while distinguishing between different medical conditions. Herein, a method is described that addresses some of the shortcomings of existing small bowel imaging techniques by using quantum dots to make microscopic changes in tissue observable to the naked eye, offering the potential for diagnosis using a non-invasive endoscopic method.

In recent years, diagnostic imaging techniques have become more advanced and less invasive or potentially harmful to the patient. For imaging of the small bowel, capsule endoscopy has become routinely used to produce images in parts of the bowel that are inaccessible by conventional endoscopes. It can be used to observe macroscopic mucosal changes, such as erosions and ulcerations, along with malignancies and sites of bleeding. Even so, its diagnostic value is limited in that it cannot provide histological data that are often necessary to distinguish between conditions with similar macroscopic presentations.

For example, Crohn's disease is chronic condition that can cause inflammation in any part of the digestive tract, leading to malabsorption and malnutrition. Complications can lead to structuring, abscess formation, perforation and fistulas. When affecting the small bowel, it commonly presents in the terminal ileum, but localised patches of inflammation can appear anywhere along the length of the gastrointestinal tract. To confirm a diagnosis of Crohn's disease, biopsy of the affected area is required to observe microscopic histological changes to the bowel mucosa and deeper layers of tissue, such as granulomatous ulcers and crypt-centred inflammation. This is required to ascertain the exact cause of ulceration, since other conditions, such as other autoimmune disorders (Behçet's disease, ulcerative jejunitis, lupus enteritis), infections (gastrointestinal tuberculosis, Yersinia enterocolitica), and use of non-steroidal anti-inflammatory drugs can result in similar macroscopic presentations, but the incorrect treatment could potentially have fatal consequences. However, biopsy of the small bowel distal to the duodenum but proximal to the ileo-caecal region is challenging, as this lies beyond the range of standard endoscopes. Techniques such as push endoscopy and double balloon enteroscopy can potentially view and biopsy the entire small bowel, but are only available at specialist centres and are not routinely used as they are considered uncomfortable procedures requiring prolonged sedation.

### Quantum Dots for Diagnostic Imaging

Quantum dots (QDs) are nanoparticles of semiconductor material with diameters typically between 2 - 10 nm. QDs display quantum confinement effects, such that their semiconductor band gap is determined by the particle size; the band gap increases as the nanoparticle diameter decreases. Upon photo-irradiation, QDs down-convert light, fluorescing at a wavelength (colour) determined by the nanoparticle size. Thus, the photoluminescence from quantum dots of a given semiconductor material can be "tuned" by manipulating the particle size. Photoluminescence in the visible region of the electromagnetic spectrum is known for a range of quantum dot materials, including those based on II-VI semiconductors, such as CdSe, and III-V semiconductors, including InP.

QDs can be prepared in colloidal solutions to yield nanoparticles with a surface that is passivated ("capped") with organic ligands. The surface ligands facilitate the processing of QDs, for example into solutions. Techniques have been developed to modify the surface passivation of QDs to render them compatible with aqueous systems. One potential application of QDs is diagnostic imaging *in vivo.*

One of the limitations of capsule endoscopy techniques in the prior art is that biopsies cannot be taken to assess microscopic changes in the lining of the bowel that are indicative of certain medical conditions. If some of the microscopic differences used to distinguish between different inflammatory conditions affecting the small bowel can be labelled, *e.g.* if their cellular compositions are different, by using different coloured QDs conjugated with specific functionalities to bind to distinct types of tissue, it may be possible to macroscopically observe some of the microscopic pathological changes in a disease *via* photoluminescence.

Methods for the use of QDs for diagnostic in the prior art are usually limited to the detection of a single specific biological entity. Edgar *et al.* proposed the use of different coloured QDs, each colour bound to a different type of phage, for the detection of multiple bacterial strains simultaneously. [R. Edgar, M. McKinstry, J. Hwang, A.B. Oppenheim, R.A. Fekete, G. Giulian, C. Merril, K. Nagashima and S. Adhya, Proc. Natl. Acad. Sci., 2006, 103, 4841]

Since transit times are often greatly increased in GI disorders, smaller particles are more likely to be absorbed than larger particles. Studies into the effect of particle size on absorption have been conducted to evaluate the potential of nanoparticles as drug carriers. Research into the effect of particle size on the uptake of fluorescent polystyrene particles (0.1, 1, and 10 µm) in rats with chemically-induced ulcerative colitis (affecting the large bowel) found that the uptake of all particle sizes was significantly enhanced in inflamed tissue, with a 6.5-fold increase in percentage particle binding for the 0.1 µm particles compared to the 10 µm particles. [A. Lamprecht, U. Schäfer and C.-M. Lehr, Pharm. Res., 2001, 18, 788] This suggests that QDs would be readily absorbed by inflamed tissue in the small bowel. Further, for bowel imaging, a contrast agent comprising QD biomarkers may therefore be better absorbed than one comprising bulk fluorescent particles. Consequently, this may allow for relatively smaller doses of a QD contrast agent, which may in turn lead to fewer side-effects.

### SUMMARY

The disclosed methods overcome limitations in the capsule endoscopy technique for visualization of the small bowel by making microscopic changes in the bowel visible to the naked eye *via* QD-analyte labelling techniques. In those patients for whom capsule endoscopy is an appropriate imaging technique, but where conventional endoscopic methods have failed to provide biopsies of the sites of inflammation, the present method may help to distinguish between differential diagnoses and to allow the most appropriate treatment option to be decided.

The capsule endoscope includes a camera and a flashing light source. Images of the small bowel are taken periodically, usually several images per second over the course of eight or more hours. The images are recorded by a series of sensors built into a belt around worn around the patient's waist. The recording is viewed after the examination by a physician, to look for any abnormalities. In the present disclosure, the light source is optimally a monochromatic LED, for example emitting blue light that could act as the excitation source for the functionalised QDs. In cases where there are differential diagnoses with distinct histopathological features, by producing a contrast agent that contains two or more different coloured QD markers to label different types of cells, it may be possible to distinguish between different conditions by the colour(s) the bowel wall fluoresces during capsule endoscopy.

Take, for example, two conditions, X and Y. Macroscopically, X and Y are almost indistinguishable. Condition X is autoimmune, requiring treatment with immunosuppressive medication such as steroids. Condition Y, on the other hand, is caused by a bacterial infection, requiring antibiotic therapy. Administration of antibiotics in condition X may do little damage other than delaying treatment and allowing the disease to progress, whereas administration of corticosteroids in condition Y would further suppress the body's ability to fight the infection, which could prove fatal. It is therefore essential to ascertain the nature of the disease prior to treatment.

There are several microscopic distinguishing features between conditions X and Y:
1. Inflamed tissue is typically located in crypt cells in condition X, but not in condition Y.
2. Acid-fast bacilli are observed for condition Y, but not X.

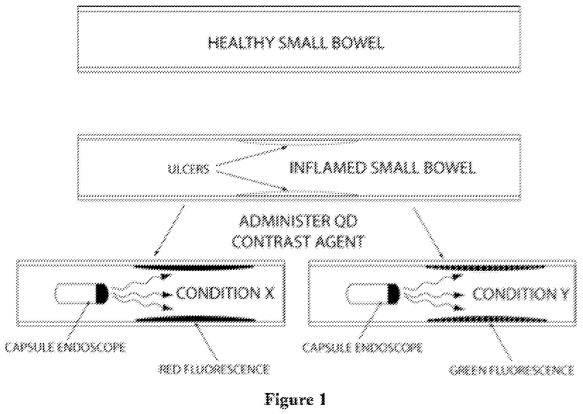

In embodiments of the disclosed methods a contrast agent is administered orally prior to a capsule endoscopy. In the current example, the contrast agent could comprise red-emitting QDs conjugated with moieties that can bind to inflammatory markers in crypt cells, and green-emitting QDs conjugated to a functionality that can bind to the bacterium causing condition Y. Those labelled QDs that are conjugated with moieties capable of binding to the abnormalities present in the bowel wall would fluoresce upon illumination by the capsule endoscope, whereas the inappropriately conjugated QDs would pass through the digestive system without adhering to bowel wall, so would not fluoresce. Simply observing whether the inflamed tissue fluoresces red or green, as illustrated in **Figure 1**, confirms the diagnosis.

In cases with established diagnosis but where a patient is failing to respond to treatment, the QD imaging technique could also be applied to assess the potential response to more potent therapeutic treatments. Again taking the example of Crohn's disease or systemic, immune-driven conditions manifesting in the small bowel (such as lupus enteritis or Behçet's disease), a relatively new class of drug treatment is monoclonal antibody therapy. These include anti-tumour necrosis factor alpha (anti-TNF-*α*) drugs, *e.g*. infliximab and adalimumab, and alpha-4 integrin inhibitors such as natalizumab. Their methods of action are different; anti-TNF-α targets the cytokine TNF-*α* that can be expressed during acute phase reactions resulting in an inflammatory response, while *α*-4 integrin mediates the attachment between a cell and the surrounding tissue, which is believed to be required for white blood cells to migrate into organs. During an inflammatory response, *α*-4 integrin may contribute to inflammation in conjunction with addressin, the endothelial cell receptor; sites of inflammation may have high levels of addressin. A wide range of monoclonal antibody therapies are now available for the treatment of cancers and autoimmune conditions.

Monoclonal antibody treatments are expensive. Two or more treatments may be required before a response to the therapy can be assessed. In the case where several monoclonal antibody treatments are available, a method to evaluate the potential of each therapy, prior to administration of the drug, could provide a substantial savings of time and money. The disclosed methods provide a way to make that evaluation by conjugating appropriate moieties (*e.g*. the protein antibody in the case of cytokines) to different coloured QDs, which are administered *in vivo* at the site of inflammation or malignancy so they can bind to any receptors present. By irradiating the tissue and observing the colour of fluorescence, it may be possible to determine which, if any, of the potential treatments is likely to be the most effective.

The imaging technique is applicable to diagnostics in other organ systems where there is an appropriate route for the administration of a contrast agent and an endoscope, but where biopsy is contraindicated.

In some aspects, the present invention includes a method for detecting a disease in a patient, the method comprising: administering to the patient a quantum dot-analyte conjugate comprising an analyte capable of binding to a marker for the disease in the patient's gastrointestinal tract, wherein the analyte is conjugated with a quantum dot having a characteristic emission wavelength; and detecting binding of the analyte to the marker by illuminating portions of the patient's gastrointestinal tract and detecting the presence of emission at the characteristic wavelength. The quantum dot may comprise indium phosphide, an alloy of indium phosphide or a doped derivative of indium phosphide. The analyte may be a cytokine antibody or an immune cell antibody, optionally the immune cell antibody is anti-TNF-α, anti-IFN-γ or canakinumab. The quantum dot-analyte conjugate may be administered orally. Alternatively, the quantum dot-analyte conjugate may be administered as a suppository or intravenously. Preferably, illuminating portions of the patient's gastrointestinal tract comprises using capsule endoscopy.

In some aspects, the present invention includes a method for discriminating between first and second possible diseases in a patient, the method comprising: administering to the patient a contrast agent comprising: (1) a first quantum dot-analyte conjugate comprising a first analyte capable of binding to a marker for the first possible disease in the patient's gastrointestinal tract, wherein the first analyte is conjugated with a first quantum dot having emission at a first characteristic wavelength; and (2) a second quantum dot-analyte conjugate comprising a second analyte capable of binding to a marker for the second possible disease in the patient's gastrointestinal tract, wherein the second analyte is conjugated with a second quantum dot having emission at a second characteristic wavelength that is different from the first wavelength; and detecting binding of either the first analyte to the marker for the first possible disease or the second analyte to the marker for the second possible disease by illuminating portions of the patient's gastrointestinal tract and detecting the presence of emission at the first or second characteristic wavelength. The quantum dot may comprise indium phosphide, an alloy of indium phosphide or a doped derivative of indium phosphide. The analyte may be a cytokine antibody or an immune cell antibody, optionally the immune cell antibody is anti-TNF-α, anti-IFN-γ or canakinumab. The quantum dot-analyte conjugate may be administered orally. Alternatively, the quantum dot-analyte conjugate may be administered as a suppository or intravenously. Preferably, illuminating portions of the patient's gastrointestinal tract comprises using capsule endoscopy.

In some aspects, the present invention includes a method for predicting a response to an immune cell antibody treatment in a patient, the method comprising: administering to the patient a quantum dot-immune cell antibody conjugate capable of binding to an immune cell in the patient's gastrointestinal tract, wherein the immune cell antibody is conjugated with a quantum dot having a characteristic emission wavelength; and detecting binding of the immune cell antibody to the marker by illuminating portions of the patient's gastrointestinal tract and detecting the presence of emission at the characteristic wavelength. The quantum dot may comprise indium phosphide, an alloy of indium phosphide or a doped derivative of indium phosphide. The analyte may be a cytokine antibody or an immune cell antibody, optionally the immune cell antibody is anti-TNF-α, anti-IFN-γ or canakinumab. The quantum dot-analyte conjugate may be administered orally. Alternatively, the quantum dot-analyte conjugate may be administered as a suppository or intravenously. Preferably, illuminating portions of the patient's gastrointestinal tract comprises using capsule endoscopy.

### Brief Description of the Drawings

**Figure 1** is a diagram illustrating the quantum dot imaging technique. For example, to distinguish between two conditions, X and Y, after administering the quantum dot contrast agent, the underlying cause of the inflammation can be distinguished by the colour the inflamed bowel wall fluoresces upon irradiation by a capsule endoscope.
**Figure 2** is a flow chart showing the process for detecting the presence of disease markers using quantum dots, as described herein.

### DESCRIPTION

Labelling of biological entities with QDs has been reported in the prior art. A given size population of QDs will fluoresce at a specific wavelength, which has been used in the detection and diagnosis of medical conditions *via in vivo* animal studies. The fluorescence wavelength (colour) of QDs depends on the particle size. The present disclosure expands on prior art imaging techniques, by combining two or more size populations of QDs, each population (colour) being labelled with moieties that will selectively bind to a disease marker that is present in one condition but is not observed in the condition(s) that are detectable using the other size population(s) of labelled QDs. By combining the technique with capsule endoscopic imaging, the labelled biological entities can be observed by their fluorescence upon illumination by the endoscopic light source. The technique offers the potential to distinguish between two or more differential diagnoses by the colour that the tissue fluoresces upon illumination. When combined with clinical findings (such as symptoms), this may enable patients to be diagnosed where the location of their condition has previously proved challenging to biopsy, such as areas of the small bowel beyond the reach of standard endoscopes.

**Table 1 summarizes some inflammatory conditions that can affect the small bowel and the histological presentations used to assist in making a diagnosis when a biopsy is taken. Where applicable, strategies that could potentially be developed to label these histological observations with QDs, within the scope of the present method, are included.**

| **Table 1: Inflammatory conditions and diagnostic strategies.** | | | |
|---|---|---|---|
| **CONDITION** | **HISTOLOGICAL OBSERVATIONS** | **QD LABELLING STRATEGY (Marker)** | **REF.** |
| **CROHN'S DISEASE (CD)** | small granulomas | granulomas - collection of macrophages - label *e.g.* with cytokine antibodies or CD11b antibody | 1, 2 |
| | no caseation | *Mycobacterium* wouldn't be present - no fluorescence in the presence of a mycobacterium-specific QD label | 3, 4 |
| | deep ulceration | n/a - observed macroscopically | |
| | inflammation targeting crypt cells | crypt inflammation is characterised by neutrophils - label with anti-myeloperoxidase | 5 |
| | mucosal changes distal to sites with granulomas | mucosal changes observable macroscopically | 1, 2 |
| | | granulomas - collection of macrophages - label *e.g.* with cytokine antibodies or CD11b antibody | |
| **GASTROINTESTINAL TUBERCULOSIS (GITB)** | acid-fast bacilli | QD bacterial labelling | 3, 4 |
| | multiple/large/ confluent granulomas | label macrophages, *e.g*. with cytokine antibodies or CD11b antibody - larger/multiple regions of fluorescence should be observed | 1, 2 |
| | caseating necrosis | caused by *Mycobacterium -* label bacteria with QDs | 3, 4 |
| | epithelioid histocytes | activated macrophages - label *e.g.* with cytokine antibodies or CD11b antibody | 1, 2 |
| **BEHÇET'S DISEASE (BD)** | vasculitis involving small/medium-sized vessels | | |
| | lymphocytic infiltrate | labelling of lymphocytes - conjugate to amine-reactive B220 antibody. | 2 |
| | dense perivascular infiltrate | inflammatory cells located around blood vessels - observable by labelling of leukocytes | 1,2,5 |
| **CONDITION** | **HISTOLOGICAL OBSERVATIONS** | **QD LABELLING STRATEGY (Marker)** | **REF.** |
| **ULCERATIVE JEJUNITIS (UJ)** | villous atropy | n/a - observed macroscopically | |
| | crypt hyperplasia | | |
| | epithelial lymphocytes | labelling of lymphocytes - conjugate to amine-reactive B220 antibody. | 2 |
| | eosinophil and plasma cell infiltration of the lamina propria | eosinophils develop in the presence of interleukin-3 and - 5 - label with QD-conjugated IL-3 or IL-5 antibodies | 6 |
| | | biopsy may be required to confirm location of eosinophils | |
| | enterocyte pleomorphism | | |
| | fibrovascular granulation tissue between crypt bases and muscularis mucosae | | |
| **LUPUS ENTERITIS (LE)** | eosinophils in deeper areas of the lamina propria between the tip of the mucosal glands and muscularis mucosa | eosinophils develop in the presence of interleukin-3 and - 5 - label with QD-conjugated IL-3 or IL-5 antibodies | 6 |
| | | biopsy may be required to confirm location of eosinophils | |
| | eosinophils and exocytosis in the mucosal glandular epithelium | eosinophils develop in the presence of interleukin-3 and - 5 - label with QD-conjugated IL-3 or IL-5 antibodies | 6 |
| | | biopsy may be required to confirm location of eosinophils | |

### Detection of Bacteria using Quantum Dots

QDs can be used for the detection of bacteria, *e.g. Mycobacterium. tuberculosis* in the case of gastrointestinal tuberculosis (GITB). Edgar *et al.* proposed a method of detecting slow growing bacterial strains such as *Mycobacterium.* [R. Edgar, M. McKinstry, J. Hwang, A.B. Oppenheim, R.A. Fekete, G. Giulian, C. Merril, K. Nagashima and S. Adhya, Proc. Natl. Acad. Sci., 2006, 103, 4841] The method, which was successfully demonstrated for the detection of *E. coli,* involved engineering a phage (virus that infects bacteria) displaying a peptide that can be biotinylated, bound to its outer shell. Streptavidin-coated QDs were conjugated to the phage. In the presence of bacteria sensitive to the phage, the phage would infect the bacteria, producing progeny virions that could be biotinylated by a protein in each bacterium. This technique enabled a high degree of amplification to occur for each bacterium present, accelerating the rate at which detection could occur. Thus, by conjugating streptavidin-coated QDs to a phage that invades *M. tuberculosis*, the method can detect GITB during capsule endoscopy.

### Detection of White Blood Cells using Quantum Dots

White blood cells (WBCs), or leukocytes, are the components of the blood that act as part of the body's immune system to defend itself from attack by harmful species such as bacteria, viruses, parasites, and foreign bodies. In autoimmune diseases, the immune system fails to recognise its own tissue, instead attacking it as if the tissue were a foreign species. High levels of WBCs are typically observed in and around inflamed tissue, though one or more types of WBC may be characteristically expressed by a particular condition. For instance, in conditions affecting the small bowel, high levels of macrophages and neutrophils (characteristic of granulomas and crypt inflammation) may be expressed in Crohn's disease, while over-expression of eosinophils is typical of lupus enteritis.

Activated white blood cells express certain proteins or enzymes, many of which have a known antibody. By conjugating QDs to the appropriate antibody, the method can be developed to detect high levels of specific types of WBCs *in situ* from the colour that they fluoresce.

As summarized in **Table 1**, granulomas are observed of a number of small bowel conditions. Granuloma size and distribution from biopsy sites are often used to differentiate between diagnoses. Since granulomas are collections of macrophages, one method for their detection would be *via* macrophage labelling. Certain cytokines (cell signaling proteins), *e.g.* TNF-*α* and IFN-γ are secreted by macrophages. Monoclonal antibody therapies, targeting cytokines, have recently generated high interest, and as such a number of cytokine antibodies are commercially available. Conjugation of appropriate cytokine antibodies to QDs could be exploited as a method of detection of macrophages *in vivo.*

Yuan *et al.* conjugated human anti-rabbit TNF-*α* antibody to CdTe QDs by forming an acrylamide bond between carboxyl groups capping the QDs and amino groups of the TNF-*α* antibody. [L. Yuan, X. Hua, Y. Wu, X. Pan and S. Liu, Anal. Chem., 2011, 83, 6800] Prior to this, the CdTe QDs were implanted onto the surface of polymer-functionalised silica nanospheres. Once conjugated with the cytokine antibodies, the QD-polymer functionalised silican nanosphere labels were used to detect TNF-*α* antibodies *via* electrochemiluminescence and square-wave voltammetry measurements.

Integrin *α*_{M} is a protein expressed by macrophage cells. The CD11b antibody targets macrophage cells that express integrin *α*_{M}. Jennings *et al.* used fluorescent CD11b-nanoparticle conjugates to detect macrophage cells in mouse spleen tissue *in vitro.* [T.L. Jennings, R.C. Triulzi, G. Tao, Z.E. St. Louis and S.G. Becker-Catania, Sensors, 2011, 11, 10570]. CD11b is sulphydryl-reactive, so was conjugated with maleimide-capped QDs *via* a thioether bond between sulphydryl groups, formed by reduction of disulphide links on the antibody, and the maleimide ligand. In the same publication, leukocyte detection was also reported *via* QD conjugation to an amine-reactive antibody, B220, which targets B-cells (lymphocytes). Conjugation was achieved by first modifying the antibody with a heterobiofunctional crosslinking molecule that targets functionalities commonly found in proteins, forming a hydrazine functionality that would ligate to 4-formylbenzene-capped nanoparticles *via* a bis-aryl hydrazine bond.

Eosinophils, which are typically over-expressed in certain autoimmune diseases including systemic lupus erythematosus, are by definition eosinophilic, i. e. can be stained by the fluorescent dye eosin. Thus, eosinophils in biopsy samples are typically detected *via* staining with eosin. Eosinophil development occurs in the presence of interleukin-3 (IL-3) and interleukin-5 (IL-5) cytokines. As such, elevated levels of eosinophils in the GI tract, as may be observed in lupus enteritis, could potentially be detected using QDs conjugated to interleukin antibodies. A method to conjugate rabbit anti-IL-3 to colloidal gold nanoparticles for the detection of IL-3 using fluorescence immunoassay techniques has previously been described by Potůćková *et al.* [L. Potůćková, F. Frando, M. Bambousková and P. Dráber, J. Immunological Methods, 2011, 371, 38] It is therefore be possible to conjugate colloidal QDs to anti-IL-3 for the detection of IL-3, as an indicator of the presence of eosinophils, *in vivo.*

Crypt cell inflammation, as observed in Crohn's disease, is typically accompanied by over-expression of activated neutrophils. QD labelling of activated neutrophils has been described by Hoshino *et al., via* the conjugation of anti-myeloperoxidase antibodies to fluorescent nanoparticles. [A. Hoshino, T. Nagao, A. Nakasuga, A. Ishida-Okawara, K. Suzuki, M. Yasuhara and K. Yamamoto, IEEE Trans. Nanobiosci., 2007, 6, 341] The myeloperoxidase enzyme is expressed on the surface of activated neutrophils, so the technique was found to selectively detect activated neutrophils, without binding to inactivated neutrophils.

Thus, in the method described herein, by preparing a contrast agent that combines a number of different QD labels, each type of QD biomarker having a different size population of QDs and therefore a different fluorescence wavelength, it is possible to distinguish between two or more potential causes of inflammation by the colour(s) inflamed tissue in the small bowel fluoresces upon irradiation by a capsule endoscope.

### Detection of Immune Cells using Quantum Dots

Immune cells, many of which have a known antibody, are over-expressed at sites of inflammation. The type of immune cells released depends on the nature of the underlying cause of inflammation, with one or more varieties of immune cell being characteristic markers of a specific condition. Thus, labelling of immune cells using QD-immune cell antibody conjugates could be exploited as a method of their detection *in vivo.*

A further embodiment involves the employment of the QD imaging technique to assess a patient's potential response to one of more monoclonal antibody therapies. Monoclonal antibody therapies target over-expressed immune cells that are responsible for the inflammatory reaction in certain diseases with the aim of damping the body's immune response. A wide range of monoclonal antibody therapies have now been developed, many of which are licensed for the treatment of disease. As such, a large number of monoclonal antibodies are commercially available. Manipulation of the surface functionalisation of QDs such that they can be conjugated to monoclonal antibodies used in disease treatment may offer a means to detect whether a patient will respond to that treatment; if the QD-antibody conjugate binds to the site of inflammation the treatment may be of benefit, whereas if no fluorescence is observed the antibodies are unlikely to target the site of inflammation and therefore the treatment may not be a worthwhile option.

TNF-*α* is a cytokine that is predominantly produced by activated macrophages. Monoclonal antibody therapies targeting TNF-*α* include infliximab and adalimumab, both of which cost in excess of £1000 per treatment. TNF-*α* has previously been labelled with QDs. [L. Yuan, X. Hua, Y. Wu, X. Pan and S. Liu, Anal. Chem., 2011, 83, 6800] CdTe QD-polymer-functionalised silica nanospheres were bonded to anti-rabbit TNF-*α* antibodies, to detect TNF-*α* using electrochemiluminescence and square-wave voltammetry measurements. Similarly, the binding of anti-TNF-*α* antibodies to the surface of QDs or QD polymer beads can be used to detect TNF-*α in vivo.* This can be used as an indicator of macrophage activity, which in turn may be proportional to the size and distribution of granulomas. For assessment of a patient's potential response to anti-TNF-*α* antibody therapy, by administering a contrast agent comprising QDs conjugated with anti-TNF-*α* antibodies prior to a capsule endoscopy, an assessment as to whether high levels of TNF-*α* are being expressed at sites of inflammation, and therefore whether the patient is likely to benefit from anti-TNF-*α* antibody therapy, can be made.

Another cytokine, IFN-γ, acts as a macrophage-activating factor. Binding anti-IFN-γ to QDs could be used to detect presence of granulomas (macrophages).

The cytokine IL-1*β*, "catabolin", is produced by activated macrophages, monocytes, fibroblasts and dendric cells. Its therapeutic antibody, canakinumab, is licensed for the treatment of a number of autoimmune diseases, with further clinical trials in progress to assess its potential in the treatment of other conditions. Conjugation of QDs with canakinumab can therefore be used to detect the presence of activated macrophages, monocytes, fibroblasts and/or dendric cells at the site of inflammation, and/or to assess a patient's potential response to treatment with canakinumab.

Within the scope of the present disclosure, by way of example, a contrast agent comprising a first colour of QDs conjugated to anti-TNF-a antibodies and a second colour of QDs conjugated to canakinumab (in appropriate proportions such that their adherence to inflamed tissue is approximately equal) can be administered to a patient prior to capsule endoscopy. The patient's potential response to treatment with one or other of the therapies can hence be evaluated by the colour(s) that the inflamed bowel tissue fluoresces upon irradiation by the capsule endoscope, with the relative fluorescence intensities acting as a predictor of which, if either, of the two therapies is likely to be the most effective. The method is not restricted to the comparison of two antibody therapies; any number of QD-antibody conjugates can be incorporated into the contrast agent providing that their fluorescence wavelengths are sufficiently distinguishable by the naked eye.

### QD Preparation

For use *in vivo,* the QD contrast agent should be non-toxic and emit in the visible region of the electromagnetic spectrum. This can be achieved using many types of semiconductor material (toxic or otherwise, providing the nanoparticles are appropriately functionalised to render them non-toxic *in vivo*). For example, III-V-based QDs, such as InP-based QDs (including alloys and doped derivatives thereof), may be employed. Recent evidence claims that the cytotoxicity of InP-based QDs is reduced upon decomposition *in vivo,* suggesting that the material would be safe for use in contrast agents administered to humans. [H. Chibli, L. Carlina, S. Park, N.M. Dimitrijevic and J.L. Nadeau, Nanoscale, 2011, 3, 2553] An example of suitable commercially available QD material is CFQD® quantum dots (Nanoco Technologies, UK). In some embodiments, the QD cores are capped with one or more shell layers of a wider band gap material, which may include one or more compositionally graded alloys, to eliminate surface defects and dangling bonds, thus improving the QD optical properties. Examples include, but are not restricted to, InP/ZnS, InP/ZnS/ZnO, or InP/ZnSe₁₋ₓSₓ.

Conjugation of QDs to antibodies or other analytes depends on functionalisation of the nanoparticles with ligands containing moieties that are able to bind to accessible functionalities in the antibody. Methods to alter the surface functionality of QDs are well known in the prior art, and include ligand exchange procedures and polymerisation techniques.

If specific particle sizes are required, *e.g.* larger than the QD regime so as to adhere only to cell membranes rather than penetrating into cells, QDs can be incorporated into polymer beads. The beads can then be functionalised to conjugate to the desired analyte. Incorporating QDs into polymer beads in this fashion may also provide an effective means to achieve aqueous compatibility, as described in the applicant's co-pending US patent application 2010/0059721, which is hereby incorporated by reference in its entirety.

### Contrast Agent Preparation

After selecting two or more desired QD-analyte conjugates, a contrast agent may be prepared by mixing the labelled QDs in appropriate concentrations such that their relative affinity to their respective target cells are equal, *i.e.* for a given area of inflamed tissue, approximately equal fluorescence intensities would be observed by the naked eye regardless of the QD-analyte conjugate (colour) with which it is labelled. The observed fluorescence intensity should take into consideration the human eye's spectral response in low light intensity conditions produced by the pulse of light from a capsule endoscope. One skilled in the art will be able to develop a method to quantify the fluorescence intensity from each colour of QD-analyte conjugates using computerised software.

For imaging of the small bowel, in some embodiments the contrast agent is prepared into an oral solution to be ingested by the patient prior to the capsule endoscopy examination. However, the method of administration is not restricted to the oral route; one skilled in the art will realise that other methods of administration, such as a suppository or *intravenous* injection, may be suitable depending on the tissue or organ to be imaged. Other than the QD-analyte conjugates, the additional components of the contrast agent, which may be added to assist in the delivery of the QD fluorescent labels and/or improve the palatability of the solution, should not fluoresce in the visible region under illumination by the wavelength of light used in the capsule endoscope. All components should be non-toxic. The contrast agent should be designed such that the QD fluorescent labels remain in the small bowel for at least the time it takes to ingest the solution and perform the examination (in the region of ten hours), but should not remain in the body for substantially longer.

In the presence of the appropriate disease marker, a QD-analyte conjugate will bind to the bowel wall. Where a disease marker is not present in the bowel, the corresponding QD-analyte conjugate will not bind to the bowel wall and will be excreted.

Following administration of the contrast agent, a capsule endoscopy is performed on the patient to image the small bowel. The QD-analyte conjugate(s) that are bound to the bowel wall are detected by their fluorescence upon irradiation by the capsule endoscope, enabling the presence of one or more specific disease markers to be identified.

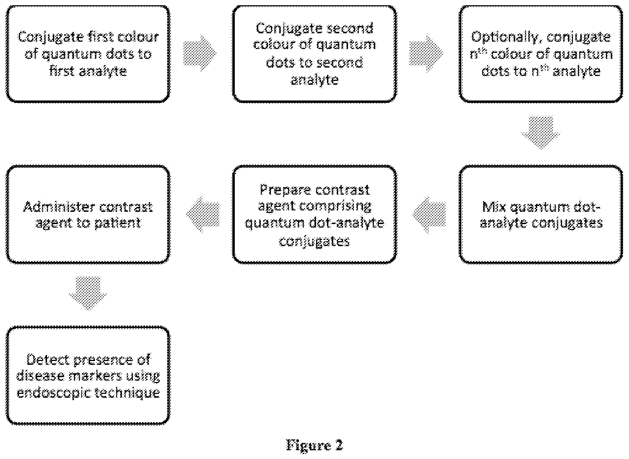

The process of using QDs to detect specific disease markers is described in **Figure 2**.

It is the aim of the present method that the QD-containing contrast agent described herein should enhance the diagnostic capabilities of the capsule endoscopy technique, by labelling specific disease markers with QDs emitting at a distinct wavelength depending on the cause of the underlying disease, to improve in the diagnosis of conditions affecting the small bowel.

Rather than just using one colour of QDs, the present method labels specific analytes with different coloured QDs which can help to distinguish between several conditions in a single endoscopy, rather than just a positive or negative answer for a single condition. Further, the precise wavelength tunability of QDs and the ability to control their conjugation by manipulating their surface chemistry offers the potential to design bespoke contrast agents based on the patient's presenting symptoms and other test results.

The imaging technique is non-invasive, unlike conventional endoscopies where there is a risk of bleeding from a biopsy site. The capsule endoscopy procedure is usually comfortable, with the patient being able to continue with daily activities throughout the test and without a recovery period afterwards. In contrast, conventional endoscopic procedures are uncomfortable, often requiring prolonged periods of sedation and a recovery period in the region of 24 hours.

When using the contrast agent to assess the potential response to monoclonal antibody therapies, the technique may provide faster access to the most effective treatment, rather than subjecting a patient to sequential courses of different treatments until a satisfactory response is achieved. This may not only save time, reducing the risk of the patient's health deteriorating further, but may also save money since monoclonal antibody therapies are highly expensive, while two or more doses may be required to assess the patient's response. Further, by providing faster access to the most effective treatment, potential side-effects of the ineffective treatment(s) are also avoided.

In summary, the present method may be of benefit to both the patient and the healthcare provider, by delivering a faster diagnosis and identification of an appropriate treatment.

### References

1. Yuan et al., Anal. Chem., 2011, 83, 6800, "Polymer Functionalized Silica Nanosphere Labels for Ultrasensitive Detection of Tumor Necrosis Factor-alpha"
2. Jennings et al., Sensors, 2011, 11, 10570, "Simplistic Attachment and Multispectral Imaging with Semiconductor Nanocrystals"
3. Edgar et al., Proc. Natl. Acad. Sci., 2006, 103, 4841, "High-Sensitivity Bacterial Detecton using Biotin-Tagged Phage and Quantum-Dot Nanocomplexes"
4. Liandris et al., PLoS ONE, 2011, 6, e20026, "Detection of Pathogenic Mycobacteria Based on Functionalized Quantum Dots Coupled with Immunomagnetic Separation"
5. Hoshino et al., IEEE Trains. Nanobiosci., 2007, 6, 341, "Nanocrystal Quantum Dot-Conjugated Anti-Myeloperoxidase Antibody as the Detector of Activated Neutrophils"
6. Potůcková et al., J. Immunological Methods, 2011, 371, 38, "Rapid and Sensitive Detection of Cytokines using Functionalized Gold Nanoparticle-Based Immuno-PCR, Comparison with Immuno-PCR and ELISA"

## Claims

1. A contrast agent for use in a method for discriminating between first and second possible diseases in a patient, wherein the contrast agent comprises:
(1) a first quantum dot-analyte conjugate comprising a first analyte capable of binding to a marker for the first possible disease in the patient's gastrointestinal tract, wherein the first analyte is conjugated with a first quantum dot having emission at a first characteristic wavelength; and
(2) a second quantum dot-analyte conjugate comprising a second analyte capable of binding to a marker for the second possible disease in the patient's gastrointestinal tract, wherein the second analyte is conjugated with a second quantum dot having emission at a second characteristic wavelength that is different from the first wavelength; and
wherein the method comprises administering the contrast agent and detecting binding of either the first analyte to the marker for the first possible disease or the second analyte to the marker for the second possible disease by illuminating portions of the patient's gastrointestinal tract and detecting the presence of emission at the first or second characteristic wavelength,
wherein illuminating portions of the patient's gastrointestinal tract comprises using capsule endoscopy.

2. A contrast agent as recited in Claim 1, wherein the first and second quantum dots comprise indium phosphide, an alloy of indium phosphide or a doped derivative of indium phosphide.

3. A contrast agent as recited in Claim 1, wherein the first or second analyte is an immune cell specific antibody; optionally wherein the immune cell specific antibody is anti-TNF-α, anti- IFN-γ or canakinumab.

4. A contrast agent for use in a method for discriminating between first and second possible diseases in a patient as recited in Claim 1, wherein the contrast agent is administered orally or as a suppository or intravenously.

5. A contrast agent for use in a method for discriminating between first and second possible diseases in a patient as recited in Claim 1, wherein at least one of the first and second analytes is an antibody that binds to a marker selected from integrin α_{M}, alpha-4 integrin, lymphocytes, IL-3, IL-5, myeloperoxidase and addressin.

6. A contrast agent for use in a method for discriminating between first and second possible diseases in a patient as recited in Claim 1, wherein the first quantum dot-analyte conjugate comprises a first analyte specific for binding to a bacteria, and the second quantum dot-analyte conjugate comprises a second analyte specific for binding to an immune cell.

7. A contrast agent for use in a method for discriminating between first and second possible diseases in a patient as recited in Claim 6, wherein bacterium is a Mycobacterium.

8. A contrast agent for use in a method for discriminating between first and second possible diseases in a patient as recited in Claim 1, wherein the first quantum dot-analyte conjugate comprises a first analyte specific for binding to macrophages and neutrophils, and the second quantum dot-analyte conjugate comprises a second analyte specific for binding to eosinophils.

9. A contrast agent for use in a method for discriminating between first and second possible diseases in a patient as recited in Claim 1, wherein the first quantum dot-analyte conjugate comprises a first analyte specific for binding to IFN-γ, TNF-α, IL-1β or integrin α_{M}, and the second quantum dot-analyte conjugate comprises a second analyte specific for binding to IL-3 or IL-5.

10. A contrast agent for use in a method for discriminating between first and second possible diseases in a patient as recited in Claim 1, wherein the first quantum dot-analyte conjugate comprises a first analyte specific for binding to IFN-γ, TNF-α, IL-1β or integrin α_{M}, and the second quantum dot-analyte conjugate comprises a second analyte specific for binding to Mycobacterium.

11. A contrast agent for use in a method for discriminating between first and second possible diseases in a patient as recited in Claim 1, wherein the first quantum dot-analyte conjugate comprises a first analyte specific for binding to lymphocytes, and the second quantum dot-analyte conjugate comprises a second analyte specific for binding to IL-3 or IL-5.

12. A contrast agent for use in a method for discriminating between first and second possible diseases in a patient as recited in Claim 1, wherein the method distinguishes between several conditions in a single endoscopy procedure.

13. A contrast agent for use in a method for discriminating between first and second possible diseases in a patient as recited in Claim 1, wherein the method determines which of a group of potential monoclonal antibody treatments is likely to be the most effective.

14. A contrast agent for use in a method for predicting a response to an immune cell specific antibody treatment in a patient, the method comprising:
administering to the patient a quantum dot-immune cell antibody conjugate capable of binding to an immune cell in the patient's gastrointestinal tract, wherein the immune cell specific antibody is conjugated with a quantum dot having a characteristic emission wavelength;
detecting binding of the immune cell specific antibody to the marker by illuminating portions of the patient's gastrointestinal tract and detecting the presence of emission at the characteristic wavelength; and
wherein illuminating portions of the patient's gastrointestinal tract comprises using capsule endoscopy.

15. A contrast agent for use in a method for predicting a response to an immune cell specific antibody treatment in a patient as recited in Claim 14, wherein the quantum dot comprises indium phosphide, an alloy of indium phosphide or a doped derivative of indium phosphide.

16. A contrast agent for use in a method for predicting a response to an immune cell specific antibody treatment in a patient as recited in Claim 14, wherein the immune cell specific antibody is anti-TNF-α, anti-IFN-γ or canakinumab.

17. A contrast agent for use in a method for predicting a response to an immune cell specific antibody treatment in a patient as recited in Claim 14, wherein the quantum dot-immune cell antibody conjugate is administered orally or as a suppository or intravenously.
